(19) Europäisches Patentamt
European Patent Office
Office européen des brevets

(11) Veröffentlichungsnummer: **0 591 773 A1**

# (12) EUROPÄISCHE PATENTANMELDUNG

(21) Anmeldenummer: **93115358.9**

(51) Int. Cl.5: **C07C 257/06**, C07D 211/84

(22) Anmeldetag: **23.09.93**

(30) Priorität: **06.10.92 DE 4233586**

(43) Veröffentlichungstag der Anmeldung:
**13.04.94 Patentblatt 94/15**

(84) Benannte Vertragsstaaten:
**AT BE CH DE DK ES FR GB GR IE IT LI LU MC NL PT SE**

(71) Anmelder: **BAYER AG**

**D-51368 Leverkusen(DE)**

(72) Erfinder: **Stoltefuss, Jürgen, Dipl.-Ing.**
**Parkstrasse 20**
**D-42781 Haan(DE)**
Erfinder: **Negele, Michael, Dr.**
**Ratinger Weg 8**
**D-42697 Solingen(DE)**

## (54) 3-Imino-3-alkoxy-propionsäurelactate und deren tautomere Acrylsäurelactate.

(57) Die Erfindung betrifft neue 3-Imino-3-alkoxy-propionsäurelactate und deren tautomere Acrylsäurelactate der allgemeinen Formel (I)

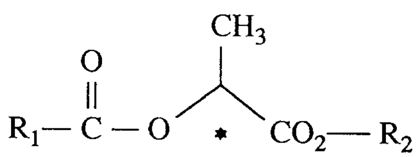

(I)

in welcher $R^1$ und $R^2$ die in der Beschreibung angegebene Bedeutung haben, Verfahren zu ihrer Herstellung und ihre Verwendung als Zwischenprodukte bei der Synthese von 2-Amino-substituierten-1,4-dihydropyridinen.

EP 0 591 773 A1

Die Erfindung betrifft neue 3-Imino-3-alkoxy-propionsäurelactate und deren tautomere Acrylsäurelactate, Verfahren zu ihrer Herstellung und ihre Verwendung als Zwischenprodukte bei der Synthese von 2-Amino-substituierten-1,4-dihydropyridinen.

Iminoether bzw. Imidoester sind bereits bekannt [vgl. S.A. Glickmann, A.C. Cope, J. Am. Chem. Soc. 67 (1945), 1017].

Die vorliegende Erfindung betrifft neue 3-Imino-3-alkoxy-propionsäurelactate und deren tautomere Acrylsäurelactate der allgemeinen Formel (I)

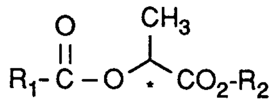

(I)

in welcher

$R^1$ für einen tautomeren Rest der Formel steht,

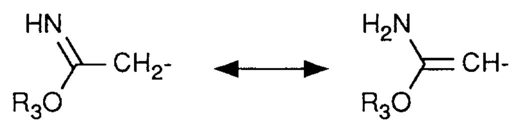

worin

$R^3$ Aryl mit 6 bis 10 Kohlenstoffatomen, geradkettiges oder verzweigtes Alkyl, Alkenyl, Alkadienyl oder Alkinyl mit jeweils bis zu 10 Kohlenstoffatomen bedeutet, die gegebenenfalls 1- oder 2-fach gleich oder verschieden durch Halogen, Hydroxy, Carboxy, Cyano, Nitro oder durch geradkettiges oder verzweigtes Alkylthio, Alkoxy, Alkoxycarbonyl, Acyl oder Acyloxy mit jeweils bis zu 8 Kohlenstoffatomen oder durch Phenoxy oder Phenyl substituiert sind, wobei die letzteren ihrerseits bis zu 2-fach gleich oder verschieden durch Halogen oder durch geradkettiges oder verzweigtes Alkyl oder Alkoxy mit jeweils bis zu 6 Kohlenstoffatomen substituiert sein können,

$R^2$ die oben angegebene Bedeutung von $R^3$ hat und mit dieser gleich oder verschieden ist

und deren Salze, freie Basen und reinen Enantiomere.

Salze sind Salze der erfindungsgemäßen Verbindungen mit anorganischen oder organischen Säuren. Bevorzugt werden Salze mit anorganischen Säuren wie beispielsweise Salzsäure, Bromwasserstoffsäure, Phosphorsäure oder Schwefelsäure.

Die erfindungsgemäßen Verbindungen existieren in stereoisomeren Formen, die sich entweder wie Bild und Spiegelbild (Enantiomere), oder die sich nicht wie Bild und Spiegelbild (Diastereomere) verhalten. Die Erfindung betrifft sowohl die Antipoden als auch die Racemformen sowie die Diastereomerengemische. Die Racemformen lassen sich ebenso wie die Diastereomeren in bekannter Weise in die stereoisomer einheitlichen Bestandteile trennen.

Beispielhaft soll dies unter Rückbezug auf die allgemeinen Formel (I) an den Resten der Formeln (II) und (IIa) erläutert werden:

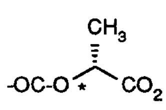

(R) (II)

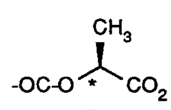

(S) (IIa)

Bevorzugt sind Verbindungen der allgemeinen Formel (I),

in welcher

$R^1$ für einen tautomeren Rest der Formel

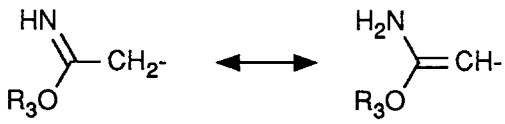

steht,
worin

$R^3$ Phenyl, Naphthyl, geradkettiges oder verzweigtes Alkyl oder Alkenyl mit jeweils bis zu 10 Kohlenstoffatomen bedeutet, die gegebenenfalls durch Halogen, Hydroxy, Carboxy, Cyano oder durch geradkettiges oder verzweigtes Alkylthio, Alkoxycarbonyl, Alkoxy, Acyl oder Acyloxy mit jeweils bis zu 6 Kohlenstoffatomen, Phenoxy oder Phenyl substituiert sind,

$R^2$ die oben angegebene Bedeutung von $R^3$ hat und mit dieser gleich oder verschieden ist

und deren Salze, freie Basen und reinen Enantiomere.

Besonders bevorzugt sind Verbindungen der allgemeinen Formel (I), in welcher

$R^1$ für einen tautomeren Rest der Formel

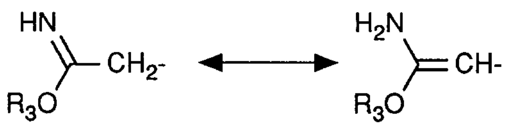

steht,
worin

$R^3$ Phenyl oder geradkettiges oder verzweigtes Alkyl mit bis zu 8 Kohlenstoffatomen bedeutet, die gegebenenfalls durch Fluor, Chlor, Hydroxy, Carboxy, Cyano oder durch geradkettiges oder verzweigtes Alkoxycarbonyl, Alkoxy oder Acyloxy mit jeweils bis zu 4 Kohlenstoffatomen substituiert sind,

$R^2$ die oben angegebene Bedeutung von $R^3$ hat und mit dieser gleich oder verschieden ist

und deren Salze, freie Basen und reinen Enantiomere.

Ganz besonders bevorzugt sind Verbindungen der allgemeinen Formel (I), in welcher

$R^1$ für einen tautomeren Rest der Formel

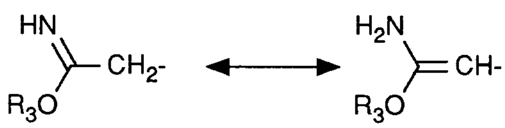

steht,
worin

$R^3$ geradkettiges oder verzweigtes Alkyl mit bis zu 4 Kohlenstoffatomen bedeutet, das gegebenenfalls durch Chlor, Fluor, Methoxy oder Ethoxy substituiert ist,

$R^2$ die oben angegebene Bedeutung von $R^3$ hat und mit dieser gleich oder verschieden ist

und deren Salze, freie Basen und reinen Enantiomere.

Außerdem wurde ein neues Verfahren zur Herstellung der erfindungsgemäßen Verbindungen der allgemeinen Formel (I) gefunden, dadurch gekennzeichnet, daß man
Verbindungen der allgemeinen Formel (III)

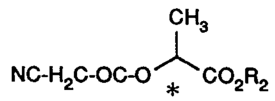

(III)

in welcher
$R^2$ die oben angegebene Bedeutung hat und bevorzugt bereits in enantiomerenreiner Form (* R oder S) vorliegt,
zunächst mit Alkoholen der allgemeinen Formel (IV)

$R^3$-OH (IV)

in welcher
$R^3$ die oben angegebene Bedeutung hat,
in inerten Lösemitteln und in Anwesenheit von Säuren umsetzt,
und im Fall der freien Basen anschließend mit Basen behandelt

Das erfindungsgemäße Verfahren kann durch folgendes Formelschema beispielhaft erläutert werden:

A*

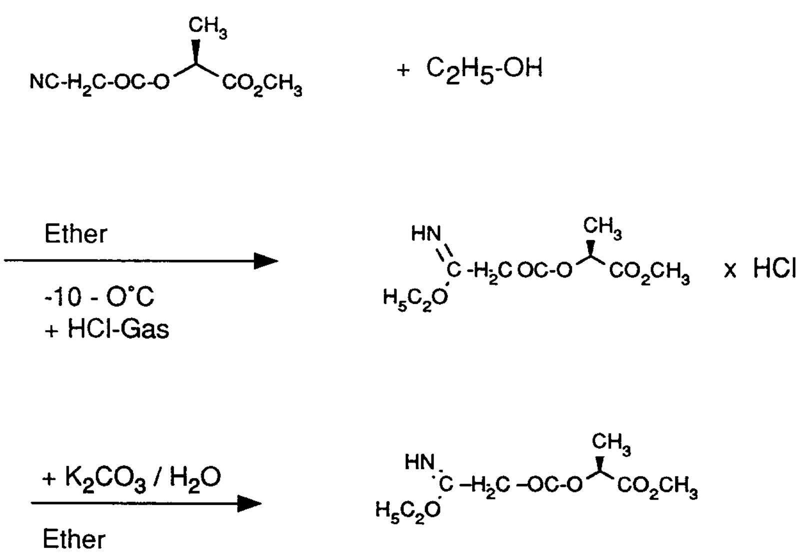


B*

*Im Folgenden bedeutet [A] die Umsetzung der Verbindungen der allgemeinen Formel (III) im mol-Mengen-Bereich, [B] die Umsetzung für den halbtechnischen Maßstab (kg-Mengen).

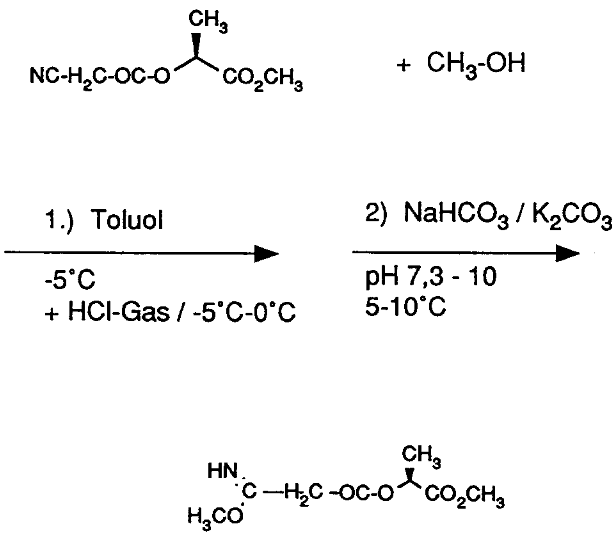


Als Lösemittel eignen sich alle organischen Lösemittel, die sich unter den Reaktionsbedingungen nicht verändern. Hierzu gehören bevorzugt Ether wie Diethylether, Diisopropylether, Methyl-tert.butylether, Dioxan, Tetrahydrofuran, Glykoldimethylether, oder Diethylenglykoldimethylether, oder halogenierte Kohlenwasserstoffe wie Methylenchlorid, Tetrachlorkohlenstoff oder Chloroform, oder Kohlenwasserstoffe wie Benzol oder Toluol.

Besonders bevorzugt ist für die Umsetzung der Verbindungen der allgemeinen Formel (III) nach [A] Diethylether, im Fall der halbtechnischen Umsetzung (kg-Bereich) nach [B] Toluol.

Als Säuren eignen sich besonders gasförmige, wasserfreie Halogenwasserstoffe, wie beispielsweise HCl oder HBr. Bevorzugt ist HCl.

Die Reaktionstemperaturen können innerhalb eines weiten Bereiches variiert werden. Im allgemeinen arbeitet man in einem Temperaturbereich von -25 ° C bis +25 ° C, bevorzugt von -10 ° C bis +10 ° C. Bei der Umsetzung A ist ein Bereich von -10 ° C bis 0 ° C, im Fall der Umsetzung B der Bereich -5 ° C bis +5 ° C, besonders bevorzugt.

Die Reaktionszeit liegt unter Einbezug der optimalen Ausbeute der erfindungsgemäßen Verbindungen der allgemeinen Formel (I) in einem Bereich von 1 bis 48 Stunden, bevorzugt bei 4 bis 24 Stunden.

Die Umsetzung kann bei Normaldruck, aber auch bei erhöhtem oder erniedrigtem Druck (z.B. 0,5 bis 30 bar) durchgeführt werden. Im allgemeinen arbeitet man bei Normaldruck.

Im allgemeinen werden 1 bis 5 mol der Alkohole der Formel (IV) und 1 - 30 mol Säure, jeweils bezogen auf 1 mol der Verbindungen der allgemeinen Formel (III) eingesetzt.

Die Freisetzung der Basen erfolgt im allgemeinen mit Alkali- oder Erdalkalicarbonaten, wie beispielsweise Natrium- oder Kaliumcarbonat.

Das erfindungsgemäße neue Verfahren zeichnet sich im Gegensatz zum Stand der Technik dadurch aus, daß zum einen aufgrund der oben aufgeführten optimierten Bedingungen die möglichen bekannten Nebenreaktionen, wie beispielsweise Spaltung der aktivierten Ester, Hydrolyse der Iminoester und/oder Folgeraktionen der Endprodukte, wie beispielsweise eine basisch katalysierte intramolekulare Esterkondensation unterdrückt werden, und andererseits die erfindungsgemäßen Verbindungen der allgemeinen Formel (I) in sehr guten Ausbeuten auf schonendem und elegantem Weg hergestellt werden können.

Die erfindungsgemäßen Verbindungen der allgemeinen Formel (I) sind wertvolle Zwischenprodukte zur Herstellung von insbesondere chiralen 2-Amino-dihydropyridinen, die in der 1,4-Dihydropyridin-Chemie von großer Bedeutung sind.

Die Alkohole der allgemeinen Formel (IV) sind bekannt.

Die Verbindungen der allgemeinen Formel (III) sind teilweise bekannt und können hergestellt werden, indem man Cyanessigsäure der Formel (V)

$NC-CH_2-CO_2H$ (V)

mit Verbindungen der allgemeinen Formel (VI)

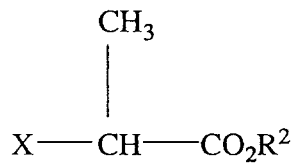

(VI)

in welcher

$R^2$ die oben angegebene Bedeutung hat

und

X für Chlor oder Hydroxy, vorzugsweise Hydroxy steht,

in einem der oben aufgeführten Lösemitteln, vorzugsweise Tetrahydrofuran und in Anwesenheit eines Dehydratisierungsmittels umsetzt.

Als Dehydratisierungsreagenzien eignen sich Carbodiimide wie beispielsweise Diisopropylcarbodiimid, Dicyclohexylcarbodiimid oder N-(3-Dimethylaminopropyl)-N'-ethylcarbodiimid-Hydrochlorid oder Carbonylverbindungen wie Carbonyldiimidazol oder 1,2-Oxazoliumverbindungen wie 2-Ethyl-5-phenyl-1,2-oxazolium-3-sulfonat oder Propanphosphonsäureanhydrid oder Isobutylchloroformat oder Benzotriazolyloxy-tris-(dimethylamino)phosphonium-hexafluorophosphat oder Phosphorsäurediphenylesteramid oder Methansulfonsäurechlorid, gegebenenfalls in Anwesenheit von Basen wie Triethylamin oder N-Ethylmorpholin oder N-Methylpiperidin oder Dicyclohexylcarbodiimid und N-Hydroxysuccinimid.

Ausgangsverbindungen

Beispiel Z1

(S)-2-Cyanoacetoxy-propionsäuremethylester

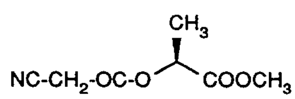

100 g (0,96 mol) (S)-Milchsäuremethylester und 81,8 g (0,96 mol) Cyanessigsäure werden in 1 l trockenem Tetrahydrofuran gelöst. In dieses Gemisch wird eine Lösung von 198,3 g (0,96 mol) Dicyclohexylcarbodiimid in 350 ml trockenem THF bei 20 °C bis 30 °C zügig zugetropft. Es wird 30 Minuten nachgerührt, vom ausgefallenem Dicyclohexylharnstoff abgesaugt und mit THF gewaschen. Das Filtrat wird eingeengt und das erhaltene Öl wird abdestilliert. Man erhält 302 g (91,8% d.Th.) eines farblosen Öles vom Siedepunkt 110-112 °C bei 0,2 mbar.

Herstellungsbeispiele

Beispiel 1

(S)-3-Imino-3-ethoxy-propionsäure-1-(methoxycarbonyl)ethylester-hydrochlorid

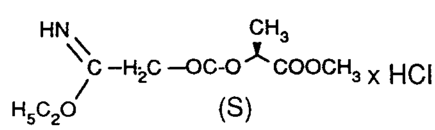

322 g (1,8 mol) der Verbindung aus Beispiel Z1 werden in 1,2 l trockenem Ether und 132 ml (2,26 mol) Ethanol gelöst. Bei -10 °C bis 0 °C wird trockenes Chlorwasserstoffgas bis zu Sättigung eingeleitet. Es wird über Nacht bei 0 °C gerührt und im Vakuum eingeengt. Man erhält ca. 476 g eines halbfesten Öles.

Beispiel 2

(S)-3-Imino-3-ethoxy-propionsäure-1-(methoxycarbonyl)-ethylester

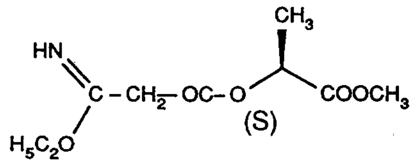

476 g der Verbindung aus Beispiel 1 werden bei 10 - 15 °C in eine Lösung von 300 g Kaliumcarbonat in 1 l Wasser gerührt, es wird mit 1,5 l Diethylether versetzt. Unter Rühren wird die wäßrige Phase durch Zugabe einer gesättigten Kaliumcarbonatlösung leicht alkalisch gestellt. Der größte Teil der gesuchten Base kristallisiert dabei aus. Es wird abgesaugt und mit Ether gewaschen. Die Etherphase des Filtrates wird von der Wasserphase getrennt, getrocknet, eingeengt, mit wenig kaltem Ether verrührt und abgesaugt. Man erhält 380 g (93% d.Th.) farblose Kristalle vom Schmp. 90-92 °C.

In Analogie zu den Vorschriften der Beispiele 1 und 2 werden die in Tabelle 1 aufgeführten Verbindungen hergestellt:

Tabelle 1:

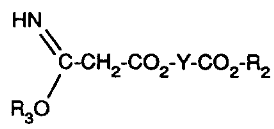

| Bsp.-Nr. | $R^3$ | Y (Enantiomer) | $R^2$ | Salz/Base | F°C | Ausbeute % d.Th. |
|---|---|---|---|---|---|---|
| 3 | $-C_2H_5$ | 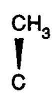 (S) | $-C_2H_5$ | Base | Öl | 85,2 % |
| 4 | $-C_2H_5$ | 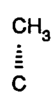 (R) | $CH_2-CH(CH_3)_2$ | HCl | 92 | 94 % |
| 5 | $-C_2H_5$ | 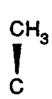 (S) | 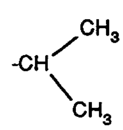 | Base | Öl | 82 % |

Beispiel 6

(S)-3-Imino-3-methoxypropionsäure-1-(methoxycarbonyl)-ethylester

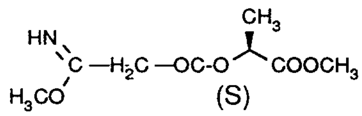

In einem 25 ltr.-Rührkessel (Emaille) werden bei -5 °C (Mantelkühlung) in 12 ltr. Toluol 8,55 kg (50 mol) (S)-2-Cyanoacetoxy-propionsäuremethylester und 2,12 kg (66,13 mol) Methanol vorgelegt. Innerhalb von 4 h werden unter Rühren ca. 4,5 kg Chlorwasserstoff (120 mol) so rasch eingeleitet, daß die Temperatur nicht über 0 °C ansteigt und die HCl weitestgehend absorbiert wird. Nach Sättigung wird über Nacht bei ca. 5 °C unter Beibehaltung der Außenkühlung nachgerührt. Zur Freisetzung des Iminoesters wird die Reaktionslösung bei 5-10 °C in eine auf pH 10 eingestellte Lösung von $NaHCO_3/K_2CO_3$ (5 kg $NaHCO_3$; 2,8 kg $K_2CO_3$ in 30 l Wasser) eingerührt. Der pH-Wert stellt sich rasch auf 7,5 ein, und muß ggfs. durch Zugabe weiterer $K_2CO_3$-Lösung korrigiert werden. Es kristallisiert der freie Iminoester aus, der nach einer Nachrührzeit von ca 1 h abgesaugt wird. Das Kristallisat wird mit 2 mal 10 ltr. Wasser nachgewaschen und über Nacht im Vakuum bei 40 °C getrocknet.
Ausbeute: 7,52 kg (74% d.Th.) Kristallisat Fp.: 81-82 °C
Aus der Toluol-Phase werden nach Einengen weitere 165 g (1,6% d.Th.) erhalten.

**Patentansprüche**

**1.** 3-Imino-3-alkoxy-propionsäurelactate und deren tautomere Acrylsäurelactate der allgemeinen Formel (I)

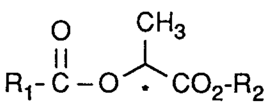

(I)

in welcher

$R^1$ für einen tautomeren Rest der Formel

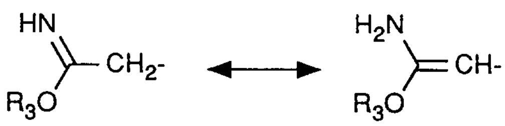

steht,
worin

$R^3$ Aryl mit 6 bis 10 Kohlenstoffatomen,
geradkettiges oder verzweigtes Alkyl, Alkenyl, Alkadienyl oder Alkinyl mit jeweils bis zu 10 Kohlenstoffatomen bedeutet, die gegebenenfalls 1- oder 2-fach gleich oder verschieden durch Halogen, Hydroxy, Carboxy, Cyano, Nitro oder durch geradkettiges oder verzweigtes Alkylthio, Alkoxy, Alkoxycarbonyl, Acyl oder Acyloxy mit jeweils bis zu 8 Kohlenstoffatomen oder durch Phenoxy oder Phenyl substituiert sind, wobei die letzteren ihrerseits bis zu 2-fach gleich oder verschieden durch Halogen oder durch geradkettiges oder verzweigtes Alkyl oder Alkoxy mit jeweils bis zu 6 Kohlenstoffatomen substituiert sein können,

$R^2$ die oben angegebene Bedeutung von $R^3$ hat und mit dieser gleich oder verschieden ist

und deren Salze, freie Basen und reinen Enantiomere.

**2.** Verbindungen der allgemeinen Formel (I) gemäß Anspruch 1,
in welcher

$R^1$ für einen tautomeren Rest der Formel

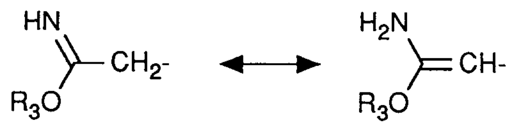

steht, worin

$R^3$ Phenyl, Naphthyl, geradkettiges oder verzweigtes Alkyl oder Alkenyl mit jeweils bis zu 10 Kohlenstoffatomen bedeutet, die gegebenenfalls durch Halogen, Hydroxy, Carboxy, Cyano oder durch geradkettiges oder verzweigtes Alkylthio, Alkoxycarbonyl, Alkoxy, Acyl oder Acyloxy mit jeweils bis zu 6 Kohlenstoffatomen, Phenoxy oder Phenyl substituiert sind,

$R^2$ die oben angegebene Bedeutung von $R^3$ hat und mit dieser gleich oder verschieden ist

und deren Salze, freie Basen und reinen Enantiomere.

**3.** Verbindungen der allgemeinen Formel (I) gemäß Anspruch 1, in welcher

$R^1$ für einen tautomeren Rest der Formel

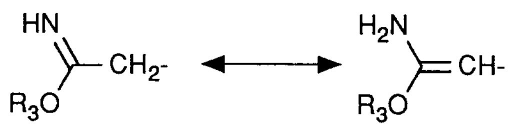

steht, worin

$R^3$ Phenyl oder geradkettiges oder verzweigtes Alkyl mit bis zu 8 Kohlenstoffatomen bedeutet, die gegebenenfalls durch Fluor, Chlor, Hydroxy, Carboxy, Cyano oder durch geradkettiges oder verzweigtes Alkoxycarbonyl, Alkoxy oder Acyloxy mit jeweils bis zu 4 Kohlenstoffatomen substituiert sind,

$R^2$ die oben angegebene Bedeutung von $R^3$ hat und mit dieser gleich oder verschieden ist

und deren Salze, freie Basen und reinen Enantiomere.

**4.** Verbindungen der allgemeinen Formel (I) gemäß Anspruch 1, in welcher

$R^1$ für einen tautomeren Rest der Formel

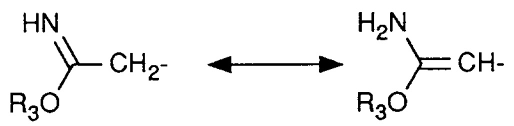

steht, worin

$R^3$ geradkettiges oder verzweigtes Alkyl mit bis zu 4 Kohlenstoffatomen bedeutet, das gegebenenfalls durch Chlor, Fluor, Methoxy oder Ethoxy substituiert ist,

$R^2$ die oben angegebene Bedeutung von $R^3$ hat und mit dieser gleich oder verschieden ist

und deren Salze, freie Basen und reinen Enantiomere.

**5.** Verfahren zur Herstellung von Verbindungen der allgemeinen Formel (I)

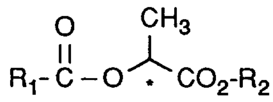 (I)

in welcher

$R^1$ für einen tautomeren Rest der Formel

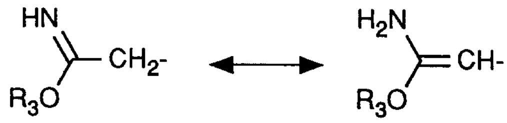

steht,

worin

$R^3$ Aryl mit 6 bis 10 Kohlenstoffatomen, geradkettiges oder verzweigtes Alkyl, Alkenyl, Alkadienyl oder Alkinyl mit jeweils bis zu 10 Kohlenstoffatomen bedeutet, die gegebenenfalls 1- oder 2-fach gleich oder verschieden durch Halogen, Hydroxy, Carboxy, Cyano, Nitro oder durch geradkettiges oder verzweigtes Alkylthio, Alkoxy, Alkoxycarbonyl, Acyl oder Acyloxy mit jeweils bis zu 8 Kohlenstoffatomen oder durch Phenoxy oder Phenyl substituiert sind, wobei die letzteren ihrerseits bis zu 2-fach gleich oder verschieden durch Halogen oder durch geradkettiges oder verzweigtes Alkyl oder Alkoxy mit jeweils bis zu 6 Kohlenstoffatomen substituiert sein können,

$R^2$ die oben angegebene Bedeutung von $R^3$ hat und mit dieser gleich oder verschieden ist

und deren Salze, freie Basen und reinen Enantiomere,

dadurch gekennzeichnet, daß man Verbindungen der allgemeinen Formel (III)

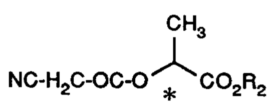 (III)

in welcher

$R^2$ die oben angegebene Bedeutung hat und bevorzugt bereits in enantiomerenreiner Form (* R oder S) vorliegt,

zunächst mit Alkoholen der allgemeinen Formel (IV)

$R^3$-OH (IV)

in welcher

$R^3$ die oben angegebene Bedeutung hat,

in inerten Lösemitteln und in Anwesenheit von Säuren umsetzt,

und im Fall der freien Basen anschließend mit Basen behandelt

**6.** Verwendung von Verbindungen der allgemeinen Formel (I) gemäß Anspruch 1 bei der Herstellung von 2-Amino-dihydropyridinen.

European Patent Office

# EUROPEAN SEARCH REPORT

Application Number

EP 93115358.9

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| P,X | EP - A - 0 515 940 (BAYER AG) * Beispiel 1, Methode A; Ansprüche * -- | 1,6 | C 07 C 257/06<br>C 07 D 211/84 |
| A | THE JOURNAL OF THE AMERICAN CHEMICAL SOCIETY, Band 67, Januar-Juni 1945 S.A. GLICKMANN et al. "Structure of beta-Amino Derivatives of the alpho,beta-Unsaturated Lactones and Esters" Seiten 1017-1020 ---- | 1,5 | |

TECHNICAL FIELDS SEARCHED (Int. Cl.5)

C 07 C 257/00
C 07 D 211/00
C 07 D 401/00

The present search report has been drawn up for all claims

| Place of search | Date of completion of the search | Examiner |
|---|---|---|
| WIEN | 30-12-1993 | HOFBAUER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document